# EUROPÄISCHE PATENTANMELDUNG

(11) **EP 2 489 370 A1**
(43) Veröffentlichungstag der Anmeldung: **22.08.2012**
(21) Anmeldenummer: 12003133.1
(22) Anmeldetag: 27.06.2008
(51) Int. Cl.: A61K 45/06, A61K 31/352, A61K 33/24, A01N 43/08, A01N 59/16, A61P 17/02, A61P 31/00

(54) **Verwendung einer synergistischen Zusammensetzung als therapeutisches Mittel oder Desinfektionsmittel**

(30) Priorität: 28.06.2007 DE 102007030103
(62) Teilanmeldung aus: 08773742.5
(71) Anmelder: Bode Chemie GmbH, 22525 Hamburg (DE); multiBIND biotec GmbH, 51105 Köln (DE)
(72) Erfinder: Krug, Barbara, Dr., 20259 Hamburg (DE); Eggerstedt, Sven, Dr., 22527 Hamburg (DE); Bloß, Richard, Dr., 25462 Rellingen (DE); Ostermeyer, Christiane, 22559 Hamburg (DE); Lisowsky, Thomas, Dr., 40789 Monheim (DE); Esser, Karlheinz, Dr., 41199 Mönchengladbach (DE); Bürger, Frank, Dr., 40597 Düsseldorf (DE)
(74) Vertreter: Kossak, Sabine

(57) **Zusammenfassung**

Gegenstand der Erfindung ist die Verwendung einer Zusammensetzung aus einem Vitamin, einem Metallion und einer oberflächenaktiven Substanz als therapeutisches Mittel oder Desinfektionsmittel. Die Zusammensetzung ist bisher aufgrund ihres effizienten DNA-Abbaus als Dekontaminationsmittel bekannt. Es konnte nun gezeigt werden, dass das Mittel eine überraschend gute Verträglichkeit zeigt bei gleichzeitiger breiter desinfizierender Wirkung und es deshalb auch in anderen Einsatzbereichen, beispielsweise zur Desinfektion oder Wundversorgung geeignet ist.

## Beschreibung

### Erfindungsgebiet

Die Erfindung betrifft neue Verwendungen einer Zusammensetzung, die zumindest aus mindestens einem Vitamin, mindestens einem Metallion und mindestens einer oberflächenaktiven Substanz besteht. Die Erfindung betrifft ferner ein therapeutisches Mittel oder Desinfektionsmittel, das mindestens einen wirksamen Bestandteil und übliche Träger- und/oder Hilfsstoffe umfasst.

### Hintergrund der Erfindung

In vielen Bereichen ist es notwendig, dass biologische und organische Verunreinigungen z.B. Proteine, DNA und Mikroorganismen vollständig von einer Oberfläche entfernt werden. Hierzu werden häufig sogenannte Dekontaminationsmittel eingesetzt, die Kontaminationen von Proteinen und Nukleinsäuren degradieren und entfernen. Es ist bekannt, dass nicht nur die Mikroorganismen selbst sondern auch einzelne DNA-Moleküle noch eine Wirkung zeigen und somit zu Infektionen führen oder Infektiösität und Pathogenität der Mikroorganismen verstärken können. Diese müssen deshalb im Wege der DNA-Dekontamination beseitigt werden, damit eine vollständige Zerstörung oder Inaktivierung der genetischen Information erfolgt. Für eine effiziente Dekontamination ist dabei notwendig, dass die freien DNA-Moleküle modifiziert, denaturiert oder degradiert werden. Die besonders wirksame Dekontamination erfolgt, wenn ein möglichst vollständiger DNA-Abbau erfolgt. Bekannte Dekontaminationslösungen enthalten häufig aggressive chemische Substanzen. So werden in Mitteln für den DNA-Abbau zur Oberflächenreinigung beispielsweise Natriumhypochlorit oder Mischungen aus Tensiden mit Phosphorsäure oder Natriumhydroxid oder Natriumazid eingesetzt. Diese aggressiven Lösungen führen teilweise zu permanenten Modifizierungen der Proteine und können partiell oxidative Schädigungen erzeugen. Sie können deshalb ausschließlich für die Dekontamination, d.h. für den DNA-Abbau auf Geräten, Instrumenten und Arbeitsoberflächen verwendet werden und zwar auch nur auf solchen, die aus Materialien bestehen, die gegen diese aggressiven Chemikalien unempfindlich sind.

Die Bekämpfung von Mikroorganismen erfolgt üblicherweise in Form von Desinfektion. Hierunter wird allgemein die effektive, irreversible Inaktivierung, Abtötung oder Entfernung von Mikroorganismen, wie Bakterien, Mykobakterien, Pilze, Hefen, Sporen, Prionen und/oder Viren von belebten und unbelebten Flächen, Geweben und Räumen verstanden. Neben einer Desinfektion ist ein vollständiger DNA-Abbau, insbesondere zur Vermeidung von Resistenzen wünschenswert. Gerade in Bereichen der Wundversorgung und bei vielen Desinfektionsanwendungen, wäre ein vollständiger Abbau und die Inaktivierung von Proteinen, Enzymen oder Nukleinsäuren schädlicher Mikroorganismen sinnvoll. Aufgrund der bisher bekannten aggressiven Mittel zum DNA-Abbau, d.h. zur Dekontamination, ist dieses jedoch nicht möglich.

In der Wundheilung werden derzeit entweder hydroaktive Verbände aus Alginaten oder Polymerschäumen auf Basis von Polyurethan oder Faserfilamente aus Biomaterialien wie Carboxymethylcellulose oder reduzierter / oxidierter Cellulose und Kollagen bzw. deren Komposite verwendet. Diese Trägermaterialien sind meist mit Silberionen dotiert, um eine bakterizide oder bakteriostatische Wirkung zu erzielen und dabei gleichzeitig das Feuchtigkeitsmilieu in der Wunde zu stabilisieren. Die Funktionalität wird definiert über den Grad und die Menge der aufgenommenen Feuchtigkeit und des Wunddebris sowie die Formulierung der Gelbildung. Definierte Eigenschaften der Silberionenfreisetzung führen zur erwünschten Bakterizidie in Abhängigkeit von der angewendeten Technologie (Laminierung, Embedding, etc ).

Die antimikrobielle Wirksamkeit des Silbers ist seit mehr als 100 Jahren bekannt und erfährt für Wundauflagen derzeit eine gewisse Renaissance. Aus Mangel an verwendbaren Alternativen nimmt man dabei auch die Nachteile des Silbers (in Form von Silberionen) in Kauf. Die Grenzen des Einsatzes von Silber als Bakteriostatikum sind mittlerweile schon gut bekannt und betreffen folgende Punkte:
- Seine antimikrobielle Wirksamkeit ist nicht für alle Mikroorganismen gleich gut und setzt zeitlich stark verzögert ein (Oligodynamie des Silbers).
- Silberionen verlieren unter Belastung (hoher Proteingehalt im Wundsekret und hohe mikrobielle Keimrate) schnell ihre antimikrobiellen Eigenschaften, was nur mit höherer Frequenz der Verbandwechselintervalle und damit höheren anfänglichen Behandlungskosten zu kompensieren ist.
- Der Übergang von Silberionen in das Blutplasma ist bei offenen Wunden unerwünscht, da bei der Bioverfügbarkeit von Silber auch schädliche toxische Effekte auftreten.
- Eine erhöhte Silberionenkonzentration im Blut führt zur Verstärkung der Blutgerinnung und damit der Thrombose-Gefahr.
- Partikelausfällung durch Chloridanteile körpereigener Elektrolyte führt zur Argyrie. Diagnostische Verfahren, wie beispielsweise die Magnetresonanztomographie, können bei Wundpatienten, die mit Silberwundauflagen behandelt wurden, nicht risikolos durchgeführt werden, da durch Magnetisierung der Ag+Cl⁻ - Partikel eine gefährliche Gefäßperforation in den Arteriolen droht. Nur bei hoher Chloridkonzentration löst sich das Silberchlorid wieder auf, da sich Dichloroargentat bildet: AgCl + Cl^{- ↔} [AgCl₂]⁻.

Topische Antibiotika sind viele Jahre in der Wundversorgung genutzt worden, weil sie selektiv zytotoxisch sind, vorrangig die fremden Bakterienzellen in der Wunde angreifen und nur geringe Wirkungen auf menschliche Zellen haben. Die Nachteile stellen sich aber wie folgt dar:
- Eine Vielzahl an topischen Antibiotika ist nur gegen spezifische Bakterien wirksam, die Wunden sind aber meist mit verschiedensten Arten kolonisiert.
- Die Spendersysteme einiger Antibiotika eignen sich oft nur bedingt zur Unterstützung anderer Aspekte des Wundmanagements, wie beispielsweise die Ableitung von Wundsekret, dessen erhöhter Anfall häufig mit einer gesteigerten Keimbesiedlung assoziiert ist.
- Lösungen, Cremes und Salben haben nicht die Fähigkeit Wundsekret aufzunehmen oder in anderer Art und Weise zu managen.
- Zusätzlich besteht das Resistenzproblem, induziert durch eine zu häufige Nutzung der Antibiotika, so dass sie oft für eine systemische Nutzung reserviert bleiben müssen.

Topische Antiseptika haben den Vorteil, dass sie eine Breitbandwirkung haben und damit fast alle Bakterienarten bekämpfen können. Trotz weit verbreiteten Einsatzes treten gewöhnlich gegen topische Antiseptika keine bakteriellen Resistenzen auf. Einige Kliniker halten die Breitbandwirkung für einen Nachteil, da die Antiseptika nicht zwischen fremden und humanen Zellen unterscheiden. Damit stellen Sie eine potenzielle Gefahr für die Wundheilung dar. Allerdings basieren die meisten zitierten Daten zur Schädlichkeit von Antiseptika auf *in vitro* Untersuchungen und nicht auf der Analyse der Wirkung auf Zellen in ihrer natürlichen Umgebung (d.h. im Gewebe). Die potenziellen schädlichen Wirkungen der Antiseptika auf die heilende Wunde sind möglicherweise eher auf ihr Spendersystem als auf ihre chemische Wirkung zurückzuführen. Eine aktuelle *in vivo* Studie demonstrierte, dass Antiseptika nicht die Wundheilung verzögern. Das typische Spendersystem für Antiseptika besteht aus Mull und wird in der Regel täglich ein- oder zweimal neu angefeuchtet. Weil Antiseptika aber an Proteine binden, haben sie im Wundbett nur eine kurze Wirkdauer (1 bis 2 Minuten). In der Wunde kann das Antiseptikum schnell von anderen Proteinquellen (wie Blut, Serum, extrazellulärer Matrix) gebunden werden und steht dann für die Abtötung von Bakterien nicht mehr zur Verfügung. Ferner erhalt Mull kein optimal feuchtes Wundmilieu und stellt auch kein physisches Hindernis für eine Sekundärbesiedlung durch Bakterien dar.

Zusammenfassend ist festzustellen, dass beide Arten von topischen antimikrobiellen Substanzen ihre Vor- und Nachteile haben. Lokale Antibiotika sind selektiv zytotoxisch, besitzen aber nur ein enges Wirkspektrum und fördern die Entstehung von Resistenzen. Während topische Antiseptika ein breites Wirkspektrum haben und die Gefahr der Resistenzbildung wesentlich geringer ist, wirken sie nicht selektiv und haben nur eine sehr kurze Wirkdauer und ein schlechtes Spendersystem.

Aus der DE 10 2005 020 327 A 1 und der WO 2006/116983 A2, die beide Grundlage der vorliegenden Erfindung sind und deren Inhalt daher auch Bestandteil der vorliegenden Anmeldung ist, ist eine Dekontaminationslösung bekannt, die eine synergistische Mischung aus mindestens einem Vitamin, mindestens einem Metallion und mindestens einer oberflächenaktiven Substanz umfasst. Diese synergistische Mischung wird zur Behandlung von zu reinigenden Oberflächen verwendet. Die Dekontaminationslösung bewirkt eine Inaktivierung und Degradation von Proteinen und Nukleinsäuren auf den behandelten Oberflächen, wobei diese Wirkung über den gesamten pH - Bereich von 2 bis 8,5 mit im Wesentlichen gleicher Effizienz erfolgt. Dadurch, dass bei diesen vergleichsweise milden pH - Werten gearbeitet werden kann, schont diese Lösung die zu behandelnden Oberflächen. Durch Aufsprühen und/oder Verreiben der Lösung oder Einweichen in der Lösung werden Proteine und Nukleinsäuren denaturiert, solubilisiert, inaktiviert, degradiert und entfernt. Die Lösung zeigt somit einen effektiven DNA-Abbau.

Bekannterweise werden Dekontaminationslösungen jedoch lediglich zur Reinigung von unbelebten Oberflächen, wie Instrumenten und Flächen eingesetzt. Augrund ihrer Aggressivität werden sie insbesondere immer dann nicht eingesetzt, wenn sie in Kontakt mit belebten Oberflächen, wie Haut, Hände oder Schleimhaut gelangen können. Die in der DE 10 2005 020 327 A1 beschriebene Dekontaminationslösung wird daher bisher lediglich zur Behandlung, d.h. Dekontamination unbelebter Oberflächen verwendet.

### Zusammenfassung der Erfindung

Es ist Aufgabe der Erfindung, neue Anwendungsbereiche für Zusammensetzungen, die mindestens ein Vitamin, mindestens ein Metallion und mindestens eine oberflächenaktive Verbindung umfassen, zu erschließen. Es ist ferner Aufgabe der Erfindung ein therapeutisches Mittel oder Desinfektionsmittel bereitzustellen, das die genannten Nachteile topischer Antibiotika und Antiseptika vermeidet, eine gute Haut- und Materialverträglichkeit zeigt und die Wundheilung unterstützt und beschleunigt. Zudem soll ein Mittel zur Verfügung gestellt werden, das neben der Dekontamination bzw. dem DNA-Abbau eine Verbesserung der Wundbehandlung und eine Desinfektion erlaubt.

Diese Aufgabe wird erfindungsgemäß gelöst, durch die Verwendung einer Zusammensetzung, die zumindest aus mindestens einem Vitamin, mindestens einem Metallion und mindestens einer oberflächenaktiven Substanz besteht als Mittel
- zur Wundversorgung,
- zur Behandlung oder Vorbeugung von Infektionen,
- zur Desinfektion oder
- zur desinfizierenden Reinigung.

Die Verwendung verschiedener Vitamine (natürlicher Antioxidanzien) in Kombination mit Metallionen und Detergentien führt zu extrem schnellen, massiven Strangbrüchen und Modifikationen in Nukleinsäuremolekülen und Proteinen. Dieser Effekt führt zu einer effizienten Inaktivierung und Zerstörung der Erbinformation und Proteine, wodurch eine besonders effektive Dekontamination erreicht wird. Es hat sich nun gezeigt, dass bei Verwendung der Zusammensetzung aus Metallionen, Vitaminen und Detergentien neben dem DNA-Abbau überraschender Weise auch eine breite antimikrobielle, antibiotische, antivirale, levurozide und fungizide Wirkung der Zusammensetzung vorliegt. Daher ist eine Verwendung auch für desinfizierende Anwendungen bei medizinischen oder therapeutischen Anwendungen, insbesondere der Desinfektion von Wunden, der Wundbehandlung und der Bekämpfung von äußeren Infektionen der Haut, Schleimhaut oder Wunden möglich. Die Zusammensetzung tötet Keime effektiv ab und desinfiziert hierdurch Wunden, Hautbereiche und Gewebe. Die Wundheilung wird gefördert und beschleunigt, wobei die Zusammensetzung aufgrund der unbedenklichen Inhaltsstoffe für Mensch und Tier sehr gut verträglich ist.

In einer Ausführungsform erfolgt die erfindungsgemäße Verwendung der Zusammensetzung deshalb zur Wundversorgung. Besonders bevorzugt wird die Zusammensetzung erfindungsgemäß als Mittel für die Wundbehandlung und/oder Wunddesinfektion und/oder Wundreinigung verwendet. Die erfindungsgemäße Verwendung des Mittels zur Wundversorgung kann beispielsweise durch Verwendung der Zusammensetzung in Verbindung mit einer Wundauflage, vorzugsweise einem Verband, einem Pflaster und/oder einer sonstigen Wundabdeckung erfolgen.

Die Zusammensetzung ist also beispielsweise eine intelligente Systemlösung für therapeutische Medizinprodukte in der Infektionskontrolle, insbesondere antimikrobiellen Wundauflagen und -spülungen zur beschleunigten Behandlung sekundär heilender Wunden. Vorteile im Hinblick auf die beschleunigte Wundheilung sind u. a. die Infektionsprävention und die Vermeidung von Resistenzen. Dabei werden die genannten Risikofaktoren von Silberionen vermieden. Zudem ist es erfindungsgemäß möglich, ein neuartiges Spendersystem auf Basis einer Trägermatrix bereitzustellen. Dieses lässt "slow release" Retardierungen zu, indem es die Wunde in einem gewünschten Feuchtigkeitsmilieu stabilisiert. Gleichzeitig wird über eine Depotwirkung der antimikrobiellen Zusammensetzung die Wundantiseptik optimiert, ohne dabei die Wundheilung negativ zu beeinflussen.

Aufgrund der überraschenden Hautverträglichkeit kann eine erfindungsgemäße Verwendung der Zusammensetzung zudem zur Behandlung oder Vorbeugung von Infektionen, Desinfektion oder zur desinfizierenden Reinigung erfolgen.

Die Verwendung der Zusammensetzung ist in einer weiteren Ausführung deshalb sowohl bei der Haut- und Händedesinfektion, als auch der desinfizierenden Reinigung möglich. Überraschenderweise zeigt die Zusammensetzung neben dem DNA-Abbau auch eine große Wirksamkeit gegen Bakterien, Mykobakterien, Viren, Pilze, Hefen, Prionen und Sporen. Die bei Betrachtung des DNA-Abbaus der Keime unerwartete Hautverträglichkeit ermöglicht erstmalig eine Anwendung sowohl in Hautdesinfektionsmitteln als auch in Händedesinfektionsmitteln sowie in kosmetischen Haut-, Händereinigungs- und -pflegemitteln.

Die Zusammensetzung kann dabei sowohl als Mittel zur kosmetischen, zur desinfizierenden als auch als Mittel zur medizinischen oder therapeutischen Behandlung verwendet werden. In der kosmetischen Behandlung ist beispielsweise ein Einsatz in Cremes, Lotionen, Gelen oder Salben zum Auftragen auf die Haut oder in die Hände möglich. Insbesondere in Hautpflegemitteln zur Behandlung von Akne, in Hautschutzsalben oder Produkten zum Einreiben der Haut nach einer Rasur (Aftershave-Lotionen oder -Lösungen). In der therapeutischen Behandlung, die sich beispielsweise bei der Behandlung von Akne mit der kosmetischen Behandlung überschneiden kann, kann die Zusammensetzung in vorteilhafter Weise als Mittel zu Wundbehandlung verwendet werden, beispielsweise als Wundspüllösung oder Wundsalbe. Therapeutisch eignet sich die Zusammensetzung insbesondere zur Behandlung von Infektionen, beispielsweise äußeren Infektionen der Haut oder von Wunden. Prophylaktisch kann das Mittel zur Verhinderung der Übertragung von Infektionen eingesetzt werden. Bei einer Verwendung der Zusammensetzung zur desinfizierenden Reinigung kann dieses als Mittel zur hygienischen Händewaschung, Händereinigung oder Hautreinigung eingesetzt werden. Dabei wirkt die Zusammensetzung in überraschender Weise sowohl gegen Bakterien, Mykobakterien, Sporen, Prionen, Hefen und Pilze als auch gegen Viren sowie Mischinfektionen dieser Keime.

Die Zusammensetzung kann als Mittel zur medizinischen oder desinfizierenden Behandlung in Anwendungskonzentration oder als Konzentrat verwendet werden. Bei der Anwendung des Mittels als Haut- oder Händedesinfektionsmittel wird dieses üblicherweise in der Anwendungskonzentration eingesetzt. Bei einer Anwendung für die Flächen- oder Instrumenten-Desinfektion werden auch Konzentrate hergestellt, die erst am Anwendungsort auf die Anwendungskonzentration verdünnt werden. Die im Rahmen der Anmeldung benannten Konzentrationen beziehen sich dabei jeweils auf das Produkt, d. h. im Falle der Anbietung als Konzentrat auf das Konzentrat und nicht auf die verdünnte Anwendungslösung.

Die Zusammensetzung kann in besonders vorteilhafter Ausgestaltung der erfindungsgemäßen Verwendung in Verbindung mit einer Wundauflage, vorzugsweise einem Verband, einem Pflaster und/oder einer sonstigen Wundabdeckung, eingesetzt werden. Dabei kann die Zusammensetzung beispielsweise in Form einer Salbe oder eines Pulvers auf ein geeignetes Trägermaterial aufgebracht werden. Alternativ oder ergänzend kann die Zusammensetzung aber auch in eine Zwischenschicht bzw. eine in sich geschlossene Lage der Wundauflage eingebracht werden. Besonders vorteilhaft ist auch die Imprägnierung der Wundauflage, insbesondere der Schichten, die mit der Wunde in Kontakt treten, mit der Zusammensetzung.

Die Zusammensetzung kann in vorteilhafter Ausgestaltung der erfindungsgemäßen Verwendung auch in gelöster Form, vorzugsweise als Wundspüllösung oder Feuchtbestandteil einer Wundauflage eingesetzt werden.

In weiterer vorteilhafter Ausgestaltung der erfindungsgemäßen Verwendung kann die Zusammensetzung ferner in pastöser und/oder halbfester Form, vorzugsweise als Creme, Gel oder Salbe eingesetzt werden.

Besonders vorteilhafte Verwendungen der Zusammensetzung können auch in fester und/oder getrockneter Form realisiert werden, wobei die Zusammensetzung vorzugsweise als Pulver, Tablette, Granulat oder Imprägnierung eingesetzt werden kann.

In einer weiteren vorteilhaften Ausgestaltung der erfindungsgemäßen Verwendung, kann die Zusammensetzung in flüssiger und/oder gelöster Form, als Schaum, Lösung, Konzentrat, Emulsion, Suspension oder Dispersion verwendet werden.

Die verwendete Zusammensetzung enthält die Komponenten bevorzugt in folgenden Mengen:
- 1 mM bis 1000 mM Vitamin, besonders bevorzugt 1 mM bis 500 mM, weiterhin bevorzugt 1 mM bis 300 mM und insbesondere 1 mM bis 100 mM,
- 0,1 mM bis 100 mM Metallion, vorzugsweise 0,4 mM bis 50 mM, insbesondere 1 mM bis 30 mM, besonders bevorzugt 1 mM bis 10 mM,
- 0,1 Gew.-% bis 35 Gew.-% oberflächenaktive Substanz, vorzugsweise 0,2 Gew.-% bis 30 Gew.-%, insbesondere 0,5 Gew.-% bis 20 Gew.-%, ganz besonders 0,5 Gew.-% bis 15 Gew.-%.

Die erfindungsgemäß verwendete Zusammensetzung weist zudem bevorzugt einen pH-Wert im Bereich von 0,5 bis 8,5, insbesondere von 1 bis 7 und vorzugsweise von 2 bis 6 bzw. 2 bis 4,5 auf.

Die Aufgabe wird ferner durch ein therapeutisches Mittel und/oder Desinfektionsmittel gelöst, das mindestens einen wirksamen Bestandteil und übliche Träger- und/oder Hilfsstoffe umfasst, und bei dem der wirksame Bestandteil zumindest eine Zusammensetzung aus mindestens einem Vitamin, mindestens einem Metallion und mindestens einer oberflächenaktiven Substanz ist. Das erfindungsgemäße Mittel weist alle wesentlichen Vorteile der oben genannten antimikrobiellen Systeme auf, ohne dabei deren Nachteile zu besitzen und hat weiterhin den zusätzlichen Nutzen der effizienten Entfernung von Nukleinsäuren und/oder Proteinen mit Relevanz für Infektiösität und Pathogenität.

Das erfindungsgemäße Mittel ist ein Drei-Komponenten-System, wobei zwei der Komponenten in einer synergistischen Reaktion für einen effizienten Abbau von Nukleinsäuren (DNA/RNA) sorgen und die dritte Komponente diesen Wirkkomplex spezifisch an den Wirkort (Erregerzellen, vorgeschädigte Humanzellen) transportiert. Der Abbau des Erbguts führt letzten Endes zur Abtötung der Erregerzellen oder der vorgeschädigten (infizierten) Humanzellen. Ungeschädigtes humanes Gewebe wird dabei überraschenderweise von dem erfindungsgemäßen Mittel nicht angegriffen oder nachteilig beeinflusst. Gerade die Eigenschaft des erfindungsgemäßen Mittels, aufgrund des Wirkmechanismus auch gegen multiresistente Erregerstämme wirksam zu sein und keine weitere Resistenzentwicklungen zuzulassen, zeigen den therapeutischen und wirtschaftlichen Nutzen des Mittels deutlich. Das erfindungsgemäße Mittel verhindert die Entwicklung und Übertragung von Resistenzen. Dadurch erfolgt z.B. eine Reduktion und Eindämmung von multiresistenten Stämmen in chronischen Wunden und eine Vermeidung der Durchseuchung mit solchen Keimen in Krankenhäusern, Altenheimen und sonstigen Einrichtungen (MRSA-Problematik). Das erfindungsgemäße Mittel wirkt dabei auch noch bei hoher Belastung. Es erfolgt eine schnelle Abtötung von Wundkeimen in einer Wundauflage oder direkt in der Wunde. Das Problem der Oligodynamie wie bei Silberionen entfällt damit. Das erfindungsgemäße Mittel wirkt selektiv auf Erregerzellen und vorgeschädigte Humanzellen, intakte Gewebe werden dagegen nicht geschädigt. Neben dem Abbau von Nukleinsäuren zeigt das erfindungsgemäße Mittel auch eine bakterizide Wirkung, die nicht nur auf den DNA-Abbau zurückzuführen ist. Trotz bakterizider Wirkung und der Fähigkeit, Nukleinsäuren abzubauen, ist das erfindungsgemäße Mittel weder mutagen noch zytotoxisch und zeigt eine gute Verträglichkeit für Haut und Schleimhaut.

Das erfindungsgemäße Mittel ist eine Zusammensetzung, die drei Komponenten umfasst, welche allesamt gut charakterisiert sind, die chemisch und toxikologisch völlig unbedenklich sind, die auch in der Kombination kein mutagenes Potenzial aufweisen, die nicht nur die pathogenen Keime abtöten, sondern auch deren genetisches Material (DNA/RNA) zerstören, und welche gesunde menschliche Zellen (auch Schleimhautzellen) nicht schädigen. Aufgrund dieser Eigenschaften, insbesondere auch der Fähigkeit des Mittels, spezifisch das Erbgut der Erreger (DNA/RNA) abzubauen, wird das Aufkommen und Verbreiten von Resistenzen bei den Wundkeimen unmöglich gemacht

Besonders vorteilhaft ist die Tatsache, dass das erfindungsgemäße Mittel im gesamten pH-Bereich von 0,5 bis 8,5 mit im Wesentlichen gleicher Effizienz wirkt. In vorteilhafter Ausgestaltung der Erfindung ist vorgesehen, dass die Zusammensetzung einen pH-Wert im Bereich von 1 bis 7, vorzugsweise von 2 bis 6, besonders bevorzugt 2 bis 4,5 aufweist. In diesen pH-Bereichen ist das erfindungsgemäße Mittel über längere Zeiträume stabil und ermöglichen einen besonders effektiven Abbau von Nukleinsäuren.

Darüber hinaus ist im Bereich von pH 4 bis 6 die Hautverträglichkeit des erfindungsgemäßen Mittels optimal. Hierdurch ist insbesondere eine Verwendung des desinfizierenden oder medizinischen Mittels zur Haut- und Händedesinfektion möglich.

Besonders bevorzugt ist eine vorteilhafte Ausführungsform, bei der die Zusammensetzung zusätzlich ein Puffersystem mit Carbonaten und Derivaten der Bernsteinsäure, vorzugsweise jeweils in einer Konzentration von 1 mM bis 500 mM, umfasst. Bei der Verwendung dieses Puffersystems in dem erfindungsgemäßen Mittel kann der pH-Wert der Lösung, der sich aufgrund der gelösten Komponenten, insbesondere der sauren Vitamine, im stark sauren Bereich befindet, leicht bis beispielsweise in den neutralen oder schwach basischen Bereich angehoben werden, ohne dass die gelösten Metallionen ausfallen. Geeignete Systeme sind u. a. Borat-, Oxalat-, Phthalat-, Glycin-, Tatrat-, Phosphat-, Carbonat-, Citrat-, Acetatpuffer.

In einer bevorzugten Ausführungsform des erfindungsgemäßen Mittels sind die enthaltenen Vitamine bzw. deren Salze oder sauren Derivate ein oder mehrere Verbindungen und/oder deren Salze ausgesucht aus der Gruppe der wasserlöslichen Vitamine mit den Eigenschaften von Antioxidanzien, wie vorzugsweise Vitamin C, Riboflavin und Niacin. Sie werden bevorzugt in Mengen von 1 mM bis 1000 mM, insbesondere in Mengen von 1 mM bis 500 mM, bevorzugt in Mengen von 1 mM bis 300 mM, besonders bevorzugt in den Mengen 1 mM bis 100 mM eingesetzt.

In einer weiteren bevorzugten Ausführungsform des erfindungsgemäßen Mittels sind die enthaltenen Metallionen 2- und /oder 3-wertige Ionen der Metalle der 4. Periode und/oder Nebengruppen I, II und VIII des periodischen Systems der Elemente. Sie werden in Form ihrer Salze mit organischen und/oder anorganische Säuren oder Basen eingesetzt. Erfindungsgemäß bevorzugt sind ein oder mehrere Verbindungen ausgesucht aus der Nebengruppe VIII, insbesondere Eisen, Kobalt, Nickel, Kupfer oder Zink. Die Metallionen werden vorzugsweise in Mengen von 0,1 mM bis 100mM, bevorzugt 0,4 mM bis 50 mM, insbesondere in Mengen von 1 mM bis 30 mM, besonders bevorzugt in den Mengen 1 mM bis 10 mM eingesetzt.

Die erfindungsgemäß enthaltenen oberflächenaktiven Substanzen können beispielsweise anionische, nichtionische, amphotere oder kationische Tenside oder geeignete Zusammensetzungen miteinander oder untereinander sein. Insbesondere können Alkylethersulfate, Alkyl- und/oder Arylsulfonate, Alkylsulfate, Amphotenside, Betaine, Alkylamidoalkylamine, alkylsubstituierte Aminosäuren und/oder Iminosäuren, acylierte Aminosäuren und/oder Amphotensidkombinationen eingesetzt werden. Grundsätzlich sind alle Tenside geeignet. Erfindungsgemäß bevorzugt sind anionische und nichtionische Tenside. Besonders bevorzugt sind natürliche oder naturanaloge Detergentien. Sie werden vorzugsweise in Mengen von 0,1 Gew.-% bis 35 Gew.-%, vorzugsweise in Mengen von 0,2 Gew.-% bis 30 Gew.-%, insbesondere in Mengen von 0,5 Gew.-% bis 20 Gew.-% ganz besonders in den Mengen 0,5 Gew.-% bis 15 Gew.-% eingesetzt.

Die Konzentrationen beziehen sich jeweils auf die Zusammensetzung, so wie sie hergestellt wird, als therapeutisches, desinfizierendes oder kosmetisches Mittel. Die beschriebenen Konzentrationen beziehen sich somit auf die Anwendungslösung. Eine Ausnahme bilden hier lediglich die desinfizierenden Mittel zur Flächen- oder Instrumenten-Desinfektion, bei denen die oben genannten Konzentrationen sich auf das Konzentrat beziehen und nicht auf die verdünnte Anwendungslösung. Flächen- und Instrumenten-Desinfektionsmittel werden üblicherweise als Konzentrate hergestellt und vertrieben. Beim Anwender erfolgt anschließend eine Verdünnung dieser Konzentrate in Verdünnungsbereichen zwischen 0,25 Gew.-% - 20 Gew.-%, bevorzugt 0,5 Gew.-% - 15 Gew.-%, besonders bevorzugt 0,5 Gew.-% - 10 Gew.-% des ursprünglichen Konzentrates.

Das erfindungsgemäße Mittel kann zusätzlich weitere Substanzen enthalten wie beispielsweise geeignete Puffersubstanzen zur Einstellung eines definierten pH-Wertes, wie beispielsweise Tris (Tris(hydroxymethyl)amino-methan), MES (2(Morpholino)ethansulfonsäure), HEPES (2-[4-(2-Hydroxyethyl)-1-piperazinyl] ethansulfonsäure und/oder MOPS (3-(N-Morpholino)-propansulfonsäure. Diese Puffersubstanzen werden in Mengen von 1 mM bis 500 mM eingesetzt.

Eine erfindungsgemäße Wundauflage umfasst mindestens eine Trägermatrix, die mit dem erfindungsgemäßen Mittel assoziiert ist. Dabei kann das erfindungsgemäße Mittel beispielsweise in Form einer Salbe oder eines Pulvers auf die Trägermatrix aufgebracht werden. Alternativ oder ergänzend kann die Zusammensetzung aber auch in eine Zwischenschicht bzw. eine in sich geschlossene Lage der Trägermatrix der Wundauflage eingebracht werden. Besonders vorteilhaft ist auch die Imprägnierung der Trägermatrix mit dem erfindungsgemäßen Mittel. Die Trägermatrix kann beispielsweise ein Verbandmaterial, Mull, Vlies oder sonstiges für das Auflegen auf Wunden geeignetes Material sein.

### Beschreibung vorteilhafter und bevorzugter Ausführungsformen der Erfindung

Die Erfindung wird durch die folgenden Figuren, Beispiele und Tabellen beispielhaft näher erläutert:

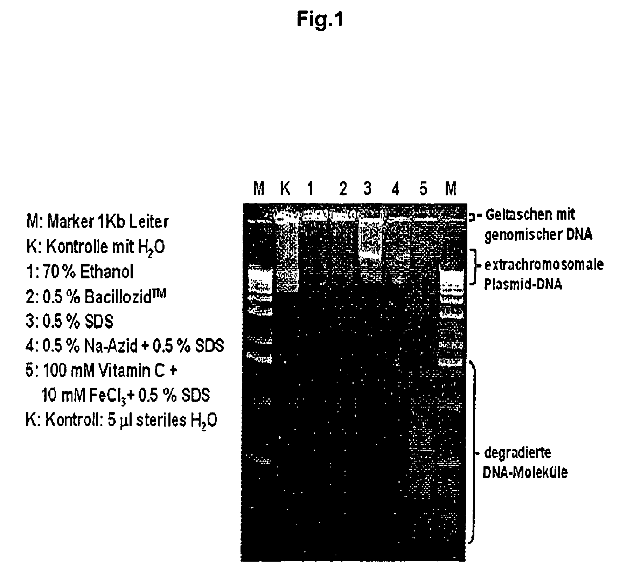

**Fig. 1** zeigt den effizienten Abbau genomischer DNA und extrachromosomaler Erbträger in Mikroorganismen durch das erfindungsgemäße Mittel (M: Marker 1 Kb Leiter; K: Kontrolle mit H₂O; 1: 70% Ethanol; 2:0.5 % BacillozidTM; 3: 0.5 % SDS; 4: 0.5 % Na-Azid + 0.5 % SDS; 5: 100 mM Vitamin C + 10 mM FeCl₃ + 0.5 % SDS; K: Kontrolle: 5 µl steriles H₂O).

**Figur 1** zeigt den effizienten Abbau genomischer DNA und extrachromosomaler Erbträger in Mikroorganismen durch das erfindungsgemäße Mittel.

Ein rekombinanter *Escherichia coli* Stamm mit einem extrachromosomalen Plasmid (YEp351) wurde aber Nacht in LB amp Medium angezogen. Je 5 µl dieser E.coli Suspension wurden mit 5µl Lysozymlösung (1 mg/ml) für 5 Minuten behandelt und dann mit 5 µl der aufgeführten Lösungen (1 - 5) für weitere 5 Minuten inkubiert. Nach Zugabe von Bromphenolblau wurden die Proben in die Geltaschen gegeben und durch Elektrophorese die DNA-Moleküle aufgetrennt. Nur in der Probe 5 mit dem erfindungsgemäßen Mittel (100 mM Vitamin C + 10 mM FeCl₃ + 0.5 % SDS) ist eine massive Degradation der DNA-Moleküle nachweisbar. Bei der Kontrolle mit sterilem Wasser (K) und bei der Probe 3 und 4 wird eine Lyse der Zellen beobachtet, so dass die extrachromosomale Plasmid-DNA freigesetzt wird und in das Gel einwandern kann. In den Proben 1 und 2 beobachtet man eine Ausfällung des Zelllysates und der DNA, so dass alle DNA-Moleküle in der Geltasche liegen bleiben.

**Tabelle 1** zeigt einen Test zur antimikrobiellen Wirkung des erfindungsgemäßen Mittels.

Frische Kulturen der angegeben Mikroorganismen wurden auf eine Zellzahl von 106 in 50 µl eingestellt und dann mit je 50 µl Wasser 70% Ethanol oder dem erfindungsgemäßen Mittel (100mM Ascorbinsäure, 10 mM FeCl₃ und 0.5% SDS) im Verhältnis 1:1 gemischt. Nach einer Inkubationszeit von 2 Minuten wurden die 100 µl Ansätze mit den Bakterien auf entsprechende Wachstumsplatten ausplattiert. Nach einer Inkubation von 1 - 3 Tagen bei 28°C (Saccharomyces cerevisiae und Candida parapsilosis) oder 37°C (Escherichia coli und Bacillus subtilis) wurde die Zahl der gewachsenen Kolonien bestimmt. In den Ansätzen mit sterilem Wasser überlebten alle Mikroorganismen. Die Ansätze mit 70 % Ethanol oder dem erfindungsgemäßen Mittel zeigten kein Koloniewachstum, was anzeigt, dass in diesen Fallen alle Mikroorganismen abgetötet wurden.

**Tabelle 1**

| Test zur antimikrobiellen Wirkung des erfindungsgemäßen Mittels. | | | |
|---|---|---|---|
| **Mikroorganismen** | **H₂O** | **70% Ethanol** | **Therapeutisches Kosmetisches Mittel** |
| Escherichia coli | 10⁶ | 0 | 0 |
| Bacillus subtilis | 10⁶ | 0 | 0 |
| Saccharomyces cevisiae | 10⁶ | 0 | 0 |
| Candida parapsilosis | 10⁶ | 0 | 0 |

### Antimikrobielle/Antivirale Wirkung:

Teststämme von S. aureus, P. aeruginosa, E. hirae und E. coli wurden in Suspensionsversuchen binnen 15 Sekunden um einen Faktor > 10⁶ reduziert. Tests mit Polioviren belegen die antivirale Wirkung des erfindungsgemäßen Mittels. In Belastungstests im Bereich der Desinfektion, bei denen die Wirkung des erfindungsgemäßen Mittels unter Zugabe von Serumprotein und/oder Schaferythrozyten getestet wurde, konnte die antimikrobielle und antivirale Wirkung des Mittels ebenfalls nachgewiesen werden. Das erfindungsgemäße Mittel wirkt also auch in Anwesenheit von Wundsekreten, Blut und anderen organischen Verschmutzungen.

Neben den oben beschriebenen Test wurden weitere Tests zur antimikrobiellen Wirkung des erfindungsgemäßen Mittels durchgeführt. Die Ergebnisse der Versuche sind in den Tabelle 2 und 3 dargestellt. Die erfindungsgemäßen desinfizierenden Mittel wurden jeweils in den beschriebenen Zusammensetzungen aus Metallsalz, Vitamin und Tenside zusammengerührt und als wässrige Lösung angesetzt. Die so erhaltenen Lösungen wurden auf die angegebenen Anwendungskonzentration (Konz.) verdünnt. Es wurden dann Tests zur Wirksamkeit des erfindungsgemäßen desinfizierenden Mittels gegen die Bakterien S. aureus, P. aeruginosa, E. coli, Proteus mirabilis durchgeführt. Die Messungen fanden bei verschiedenen Bakterienbelastungen statt und es folgte jeweils eine Bestimmung des Reduktionsfaktors nach 15 Sekunden, 30 Sekunden und 60 Sekunden.

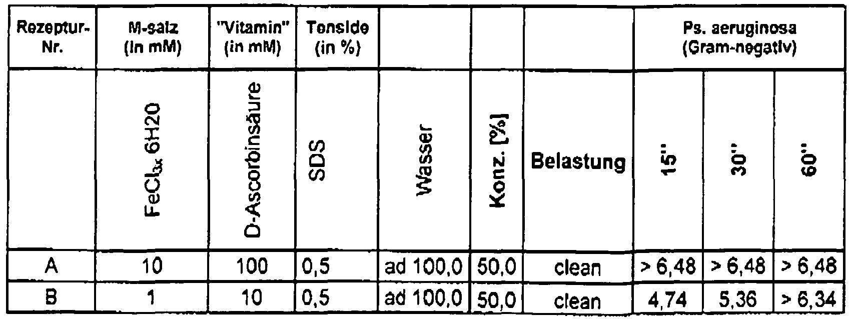

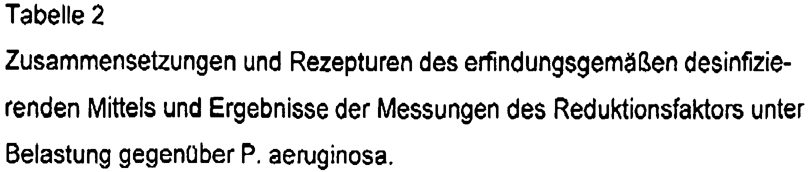

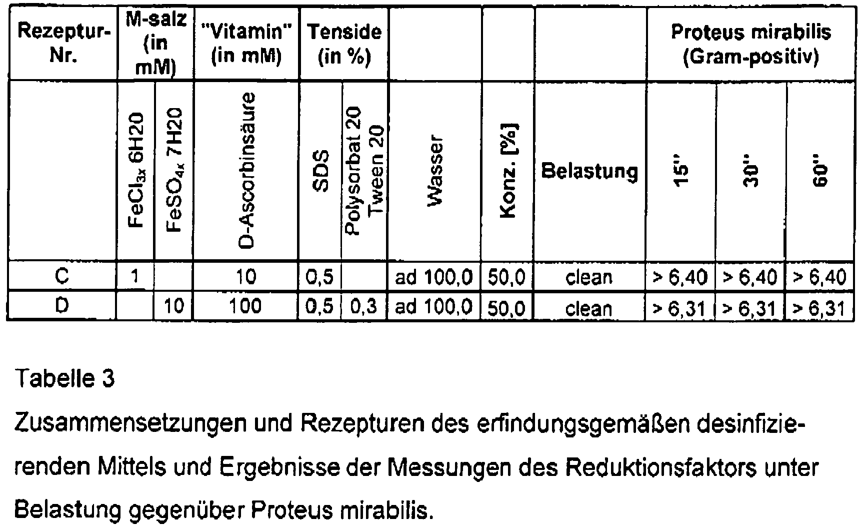

Die Messungen der antibakteriellen Wirkung des erfindungsgemäßen Mittels zeigen, dass dieses neben dem effizienten Abbau der Nukleinsäuren auch einen gute bakterizide Wirkung zeigt. Das Mittel lässt sich somit leicht sowohl zur Hautdesinfektion, als auch zur Händedesinfektion einsetzen. Aufgrund der hohen Wirksamkeit ist eine Anwendung auch bei der desinfizierenden Reinigung, wie der kosmetischen oder hygienischen Händewaschung, bei der eine niedrige Konzentration im Produkt vorliegt, möglich. Es ist somit sowohl eine Desinfektion gesunder Haut oder Schleimhaut als auch eine Desinfektion im Wundbereich oder im Bereich um eine Wunde herum oder kranker Haut oder Schleimhaut möglich.

In weiteren Test wurde zudem die antivirale Wirkung des erfindungsgemäßen Mittels untersucht. Hierzu wurden die Reduktionsfaktoren nach einer Minute, 15 Minuten und 60 Minuten gemessen. Es wurde die Wirksamkeit gegen die Viren Polyoma und Vaccinia bestimmt. Die Zusammensetzung der jeweiligen Testlösungen und die Ergebnisse der Werte sind in den folgenden Tabelle 4 und 5 wiedergegeben.

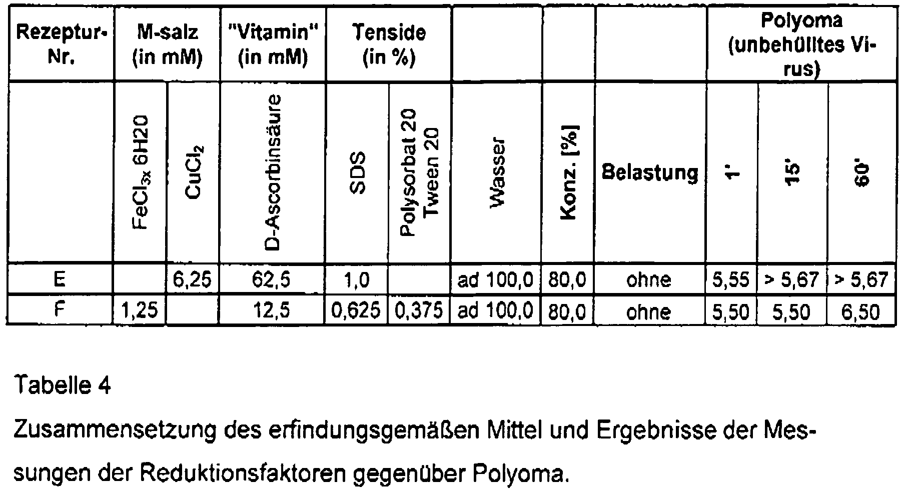

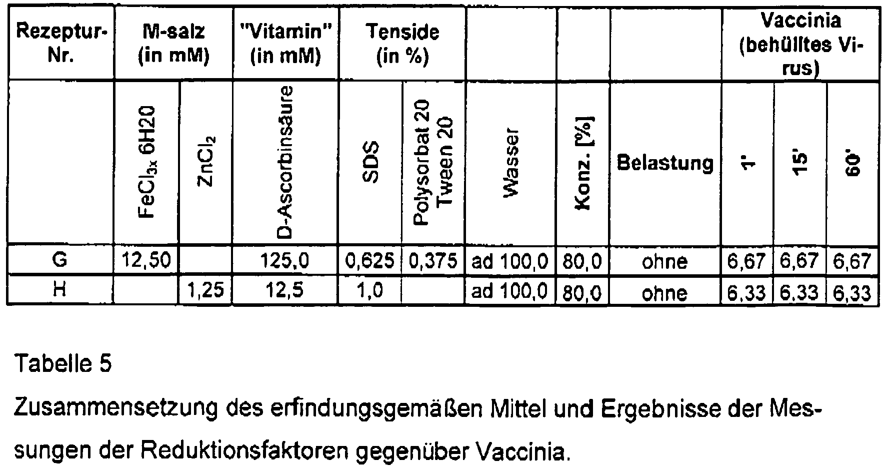

Die Tests haben ergeben, dass die erfindungsgemäßen Mittel neben dem Abbau der Nukleinsäuren und den guten antibakteriellen Wirkungen auch überraschend gute antivirale Wirkungen zeigen. So konnte gegenüber allen getesteten Viren eine sehr gute Reduktion bereits nach kurzen Einwirkzeiten beobachtet werden.

Weitere Tests haben zudem ergeben, dass auch gegenüber Pilzen und Hefen eine hohe Wirksamkeit des erfindungsgemäßen Mittels vorliegt. Zusammenfassend lässt sich somit festhalten, dass die Messergebnisse zeigen, dass neben dem Nukleinabbau die erfindungsgemäßen Mittel bei große Hautverträglichkeit auch einen große Wirksamkeit gegen Bakterien, Mykobakterien, Viren, Pilze, Hefen, Prionen und Sporen ausweisen. Das erfindungsgemäße Mittel lässt sich daher erfolgreich in Bereichen anwenden, in denen eine Belastung mit verschiedensten Keimen vorliegt und eine Reduktion verschiedener Keimarten gewünscht ist.

### Hautverträglichkeit :

Umfangreiche Daten zu den einzelnen Bestandteilen des erfindungsgemäßen Mittels zeigen deren generell gute Hautverträglichkeit. In dem erfindungsgemäßen Mittel werden ausschließlich Bestandteile bzw. Substanzen eingesetzt, die jeweils einzeln in verschiedensten Formen bereits in Kosmetika, Nahrungsmitteln, Nahrungsergänzungsmitteln, Medizinprodukten und Arzneimitteln verwendet werden. Erste Versuche mit dem erfindungsgemäßen Mittel, also der Kombination aus Vitamin, Metallion und Detergentien, ergaben ebenfalls keine ungewöhnlichen Hautveränderungen.

### Biokompatibilität:

Das erfindungsgemäße Mittel wurde in den höchsten Konzentrationen im Bruce-Ames-Test auf Mutagenität getestet. Dabei konnte kein mutagenes Potential festgestellt werden. Auch im aktivierten Bruce-Ames-Test mit Leberzellextrakten zeigte sich keine Mutagenität. Bei ersten Tests des erfindungsgemäßen Mittels am Hühnereimodell konnten ebenfalls keine schädlichen Einflüsse auf die Entwicklung festgestellt werden.

### Materialverträglichkeit:

Neben der Hautverträglichkeit ist bei der Verwendung des erfindungsgemäßen Mittels auch eine hohe Materialverträglichkeit von Bedeutung. Bei Messungen der Materialverträglichkeit gegenüber Polyethylen, Polypropylen, Polycarbonat, PMMA, Polystyrol, PTFE, PVC, Silikon, Latex und Viton konnte eine gute Materialverträglichkeit nachgewiesen werden. Eine Anwendung des desinfizierenden Mittels ist somit auch als Mittel zur Flächen- oder Instrumenten-Desinfektion möglich.

## Patentansprüche

1. Verwendung einer Zusammensetzung, die zumindest aus mindestens einem Vitamin, mindestens einem Metallion und mindestens einer oberflächenaktiven Substanz besteht als Mittel
- zur Wundversorgung,
- zur Behandlung oder Vorbeugung von Infektionen,
- zur Desinfektion oder
- zur desinfizierenden Reinigung.

2. Verwendung nach Anspruch 1, wobei die Zusammensetzung als Mittel zur Wundversorgung für die Wundbehandlung und/oder Wunddesinfektion und/oder Wundreinigung eingesetzt wird.

3. Verwendung nach Anspruch 1 oder 2, wobei die Zusammensetzung in Verbindung mit einer Kompresse oder Wundauflage, vorzugsweise einem Verband, einem Pflaster und/oder einer sonstigen Wundabdeckung eingesetzt wird.

4. Verwendung nach Anspruch 1, wobei die Zusammensetzung als Mittel zur Behandlung oder Vorbeugung von Infektionen durch Bakterien, Mykobakterien, Pilze, Hefen, Sporen, Prionen und/oder Viren eingesetzt wird.

5. Verwendung nach Anspruch 1 oder 4, wobei das Desinfektionsmittel ein Haut-, Schleimhaut- oder Händedesinfektionsmittel ist.

6. Verwendung nach Anspruch 1, wobei das Mittel zur desinfizierenden Reinigung, ein Mittel zur hygienischen Händewaschung, Händereinigung oder Hautreinigung ist.

7. Verwendung nach einem der vorhergehenden Ansprüche, wobei die Zusammensetzung das Vitamin in Mengen von 1 mM bis 1000 mM und/oder das Metallion in Mengen von 0,1 mM bis 100 mM und/oder die oberflächenaktive Substanz in Mengen von 0,1 Gew.-% bis 35 Gew.-% enthält und/oder die Zusammensetzung einen pH-Wert im Bereich von 0,5 bis 8,5 aufweist.

8. Verwendung nach einem der Ansprüche 1 bis 7, wobei die Zusammensetzung in gelöster oder aufgeschäumter Form, vorzugsweise als Wundspüllösung oder Feuchtbestandteil einer Wundauflage, eingesetzt wird.

9. Verwendung nach einem der Ansprüche 1 bis 7, wobei die Zusammensetzung in pastöser und/oder halbfester Form, vorzugsweise als Creme, Gel oder Salbe eingesetzt wird.

10. Verwendung nach einem der Ansprüche 1 bis 7, wobei die Zusammensetzung in fester und/oder getrockneter Form, vorzugsweise als Pulver, Granulat, Tablette oder Imprägnierung eingesetzt wird.

11. Verwendung nach einem der Ansprüche 1 bis 7, wobei die Zusammensetzung in flüssiger und/oder gelöster Form, vorzugsweise als Lösung, Konzentrat, Emulsion, Dispersion, Schaum oder Suspension vorliegt.

12. Therapeutisches Mittel oder Desinfektionsmittel zumindest enthaltend mindestens einen wirksamen Bestandteil und übliche Träger- und/oder Hilfsstoffe, **dadurch gekennzeichnet, dass** der wirksame Bestandteil zumindest eine Zusammensetzung aus mindestens einem Vitamin, mindestens einem Metallion und mindestens einer oberflächenaktiven Substanz ist.

13. Mittel nach Anspruch 12, **dadurch gekennzeichnet, dass** die Zusammensetzung einen pH-Wert im Bereich von 0,5 bis 8,5, insbesondere von 1 bis 7, vorzugsweise von 2 bis 6, besonders bevorzugt 2 bis 4,5 aufweist.

14. Mittel nach einem der Ansprüche 12 bis 13, **dadurch gekennzeichnet, dass** das Mittel ein Haut-, Schleimhaut-, Hände-, Flächen- oder Instrumentendesinfektionsmittel ist.

15. Mittel nach einem der Ansprüche 12 bis 14, **dadurch gekennzeichnet, dass** das Vitamin in Mengen von 1 mM bis 1000 mM, vorzugsweise in Mengen von 1 mM bis 500 mM, bevorzugt von 1 mM bis 300 mM, besonders bevorzugt in einer Menge von 1 mM bis 100 mM enthalten ist.

16. Mittel nach einem der Ansprüche 12 bis 15, **dadurch gekennzeichnet, dass** das Metallion in Mengen von 0,1 mM bis 100 mM, vorzugsweise in Mengen von 0,4 mM bis 50 mM, insbesondere in Mengen von 1 mM bis 30 mM, besonders bevorzugt in den Mengen 1 mM bis 10 mM enthalten ist.

17. Mittel nach einem der Ansprüche 12 bis 16, **dadurch gekennzeichnet, dass** die oberflächenaktive Substanz in Mengen von 0,1 Gew.-% bis 35 Gew.- %, vorzugsweise in Mengen von 0,2 Gew.-% bis 30 Gew.-%, insbesondere in Mengen von 0,5 Gew.-% bis 20 Gew.-%, ganz besonders in den Mengen 0,5 Gew.-% bis 15 Gew.-% enthalten ist.

18. Wundauflage mit mindestens einer Trägermatrix, die mit einem Mittel nach einem der Ansprüche 12 bis 17 assoziiert ist.

19. Verwendung zumindest einer Zusammensetzung aus mindestens einem Vitamin, mindestens einem Metallion und mindestens einer oberflächenaktiven Substanz zur Herstellung eines Mittels für die Behandlung von Wunden am menschlichen oder tierischen Körper.
